# EUROPEAN PATENT APPLICATION

(11) **EP 3 811 855 A1**
(43) Date of publication of application: **28.04.2021**
(21) Application number: 19823432.0
(22) Date of filing: 03.06.2019
(51) Int. Cl.: A61B 5/02, A61B 5/0285, A61B 5/1455

(54) **MEASUREMENT DEVICE, MEASUREMENT METHOD, AND MEASUREMENT PROGRAM**

(30) Priority: 22.06.2018 JP 2018118674
(71) Applicant: Kyocera Corporation, Kyoto-shi, Kyoto 612-8501 (JP)
(72) Inventor: HIRANO Asao, Kyoto-shi, Kyoto 612-8501 (JP); HIGUCHI Takeshi, Kyoto-shi, Kyoto 612-8501 (JP)
(74) Representative: Viering, Jentschura & Partner mbB Patent- und Rechtsanwälte
(86) International application number: PCT/JP2019/022026
(87) International publication number: WO 2019/244611

(57) **Abstract**

Provided is a measurement apparatus including a first measurement unit and a controller. The first measurement unit measures at least one of percutaneous oxygen saturation (SpO₂) and blood flow amount of a subject. The controller estimates health condition of the subject based on information including at least one of a measured value of SpO₂ and a measured value of blood flow amount.

## Description

### CROSS-REFERENCE TO RELATED APPLICATION

The present application claims priority to and the benefit of Japanese Patent Application No. 2018-118674 filed on June 22, 2018, the entire contents of which are incorporated herein by reference.

### TECHNICAL FIELD

The present disclosure relates to a measurement apparatus, a measurement method and a measurement program.

### BACKGROUND

A known measurement apparatus is attached to a human body to measure biological information. For example, Patent Literature 1 (PTL 1) discloses an ear-worn device that is worn on ears to detect biological information and calculates a blood flow amount state value based on the detected biological information.

### CITATION LIST

### Patent Literature

PTL 1: JP2005-192581A

### SUMMARY

### (Solution to Problem)

An aspect of a measurement apparatus includes a first measurement unit and a controller.

The first measurement unit measures at least one of percutaneous oxygen saturation (SpO₂) and blood flow amount of a subject.

The controller estimates health condition of the subject based on information including at least one of a measured value of the SpO₂ and a measured value of the blood flow amount..

An aspect of a measurement method includes a measurement step and an estimation step.

The measurement step measures at least one of percutaneous oxygen saturation (SpO₂) and blood flow amount of a subject.

The estimation step estimates health condition of the subject based on information including at least one of a measured value of the SpO₂ and a measured value of the blood flow amount.

An aspect of a measurement program causes a computer to execute following steps:
(1) a measurement step of measuring at least one of percutaneous oxygen saturation (SpO₂) and blood flow amount of a subject; and
(2) an estimation step of estimating health condition of the subject based on information including at least one of a measured value of the SpO₂ and a measured value of the blood flow amount.

### BRIEF DESCRIPTION OF THE DRAWINGS

In the accompanying drawings:
FIG. 1 is a functional block diagram illustrating a schematic configuration of a measurement apparatus according to a first embodiment;
FIG. 2 is a schematic diagram for illustrating an example of a usage state of the measurement apparatus in FIG. 1;
FIG. 3 is a flowchart illustrating an example of processing executed by a controller in FIG. 1;
FIG. 4 is a functional block diagram illustrating a measurement apparatus according to a second embodiment;
FIG. 5 is a schematic diagram for illustrating an example of a usage state of the measurement apparatus in FIG. 4;
FIG. 6 is a functional block diagram illustrating a schematic configuration of a measurement system according to a third embodiment;
FIG. 7 is a sequence diagram illustrating an example of a control procedure by a measurement system 300 in FIG. 6;
FIG. 8 is a diagram schematically illustrating an example of a cerebral blood flow amount meter;
FIG. 9 is a diagram schematically illustrating an example of a blood-pressure gauge;
FIG. 10 is a diagram schematically illustrating an example of a thermometer;
FIG. 11 is a diagram schematically illustrating an example of a state where a measuring instrument equipped with a measurement apparatus that measures a temple as a measured part is worn;
FIG. 12 is a partial cross section of the measuring instrument illustrated in FIG. 11;
FIG. 13 is a schematic external perspective view of a measuring instrument according to another embodiments;
FIG. 14 is a schematic external perspective view of the measuring instrument in FIG. 13 viewed from a different direction;
FIG. 15 is a schematic external perspective view illustrating a state where a biosensor is connected to a connector of the measuring instrument in FIG. 13;
FIG. 16 is a schematic diagram illustrating an example of a state where the measuring instrument in FIG. 13 is worn; and
FIG. 17 is a schematic diagram illustrating an example of a state where a measuring instrument according to another embodiment is worn.

### DETAILED DESCRIPTION

Embodiments will be described in detail below with respect to the accompanying drawings. When the biological information is measured by using an existing measurement apparatus, it is necessary to use a different measurement apparatus depending on the biological information to be measured. Thus, it is needed to wear a plurality of measurement apparatuses in order according to a measurement order of the biological information to be measured, which is not always convenient. In such a measurement apparatus, it is desirable to enhance the convenience in estimating the health condition of the subject. The present disclosure relates to providing a measurement apparatus, a measurement method and a measurement program that enhance the convenience in estimating the health condition of the subject. According to the present disclosure, a measurement apparatus, a measurement method and a measurement program that enhance the convenience in estimating the health condition of the subject can be provided.

### (First Embodiment)

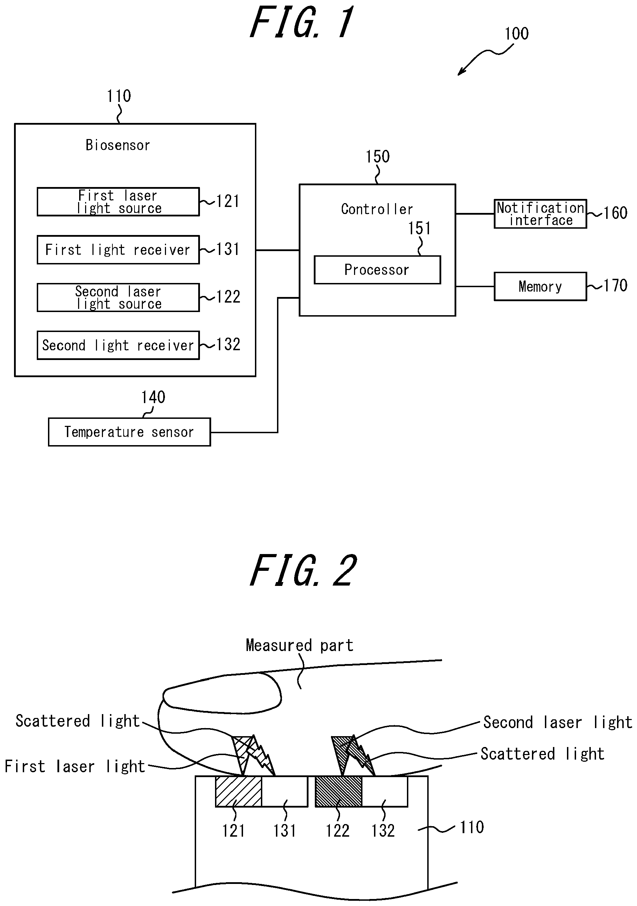

FIG. 1 is a functional block diagram illustrating a schematic configuration of a measurement apparatus 100 according to a first embodiment. The measurement apparatus 100 according to this embodiment includes a biosensor 110 constituting a first measurement unit, a temperature sensor 140 constituting a second measurement unit, a controller 150, a notification interface 160 and a memory 170.

The measurement apparatus 100 acquires biological measurement output of a subject (user) in contact with the measurement apparatus 100 by using at least one of the biosensor 110 and the temperature sensor 140, and measures the biological information based on the biological measurement output. The measurement apparatus 100 according to this embodiment can measure at least one of the oxygen saturation and the blood flow amount of the subject by using the biosensor 110. Further, the measurement apparatus 100 according to this embodiment can measure the temperature (e.g., the body temperature) at a predetermined part of the subject by using the temperature sensor 140.

The measurement apparatus 100 according to this embodiment can measure, for example, the percutaneous arterial oxygen saturation (SpO₂, S: Saturation, P: Percutaneous or Pulse Oximetry, O₂: Oxygen) as a value indicating the oxygen saturation of the subject. However, the biological information measured by the measurement apparatus 100 is not limited to the SpO₂ and the blood flow amount. The measurement apparatus 100 may measure any biological information that can be measured by using the biosensor 110. Hereinafter, SpO₂ will also be referred to simply as oxygen saturation. As a value indicating the oxygen saturation, SaO₂ (S: Saturation, a: artery, O₂: Oxygen) is used, which indicates a measured value of oxygen saturation of arterial blood. Further, SpO₂ is a method of indirectly measuring SaO₂ and, under prepared measurement conditions, both take an approximate value.

The biosensor 110 constituting the first measurement unit acquires biological measurement output from a measured part of the subject in contact with the measurement apparatus 100. The measured part is any part from which biological measurement output can be acquired. According to this embodiment, the measured part is assumed to be a finger in the description below. The measured part may be a wrist, an arm, an ear, a forehead, a neck, a back, a foot, other parts, or any combination thereof, in place of or in addition to a finger. The biosensor 110 includes a light emitter and a light receiver. In this embodiment, the biosensor 110 includes, as a light emitter, a first laser light source 121 and a second laser light source 122. In this embodiment, the biosensor 110 includes, as a light receiver, a first light receiver 131 and a second light receiver 132.

Each of the first laser light source 121 and the second laser light source 122 emits laser light with a wavelength capable of detecting a predetermined component in the blood as measuring light. Each of the first laser light source 121 and the second laser light source 122 is configured as, for example, a Laser Diode (LD). In this embodiment, a vertical cavity surface emitting laser (VCSEL) is used as a laser light source. However, a Distributed Feedback (DFB) laser or a Fabry-Perot (FP) laser can also be used.

The first laser light source 121 and the second laser light source 122 emit laser light of different wavelengths. The first laser light source 121 emits laser light of a first wavelength (hereinafter also referred to as "first laser light"). The first wavelength is a wavelength that exhibits a large difference between absorbance in hemoglobin bonded with oxygen (hereinafter, referred to as "oxygenated hemoglobin") and absorbance in hemoglobin not bonded with oxygen (hereinafter, referred to as "reduced hemoglobin"). The first wavelength is, for example, 600 nm to 700 nm, and the first laser light is so-called red light. In this embodiment, the first wavelength will be assumed to be 660 nm in the following description. The second laser light source 122 emits laser light of a second wavelength (hereinafter, also referred to as "second laser light"). The second wavelength is different from the first wavelength. The second wavelength is a wavelength that exhibits a smaller difference between absorbance in oxygenated hemoglobin and absorbance in reduced hemoglobin than that of the first wavelength. The second wavelength is, for example, 800 nm to 1000 nm, and the second laser light is so-called near infrared light. In this embodiment, the second wavelength will be assumed to be 850 nm in the following description.

The first light receiver 131 and the second light receiver 132 receive, as a biological measurement output, scattered light (detection light) of the measuring light irradiated on and scattered from the measured part. Each of the first light receiver 131 and the second light receiver 132 is configured as, for example, a Photo Diode (PD). The biosensor 110 transmits photoelectric conversion signals of scattered light received by the first light receiver 131 and the second light receiver 132 to the controller 150.

FIG. 2 is a schematic diagram illustrating an example of a usage state of the biosensor 110 in the measurement apparatus 100. As schematically illustrated in FIG. 2, the biosensor 110 measures biological information in a state where the subject causes the measured part to be in contact with a specific location (a measurement unit) on the biosensor 110. The biosensor 110 may measure the biological information in a state where the subject does not cause the measured part to be in contact with the specific location (the measurement unit) of the biosensor 110.

As schematically illustrated in FIG. 2, the first light receiver 131 receives, from the measured part, scattered light of the first laser light emitted by the first laser light source 121. The first light receiver 131 may be configured as a PD capable of detecting light of a wavelength corresponding to scattered light of first laser light (red light). As schematically illustrated in FIG. 2, the second light receiver 132 receives, from the measured part, scattered light of the second laser light emitted by the second laser light source 122. The second light receiver 132 may be configured as a PD capable of detecting light of a wavelength corresponding to scattered light of second laser light (near infrared light). In the biosensor 110, the first light receiver 131 is disposed at a position at which scattered light of laser light emitted by the first laser light source 121 can be received. Further, in the biosensor 110, the second light receiver 132 is disposed at a position at which scattered light of laser light emitted by the second laser light source 122 can be received.

Here, a relationship between the first laser light, the second laser light, and the light scattered therefrom will be described. The reduced hemoglobin easily absorbs the first laser light, which is a red light, and is less likely to absorb the second laser light, which is a near infrared light. On the other hand, the oxygenated hemoglobin is less likely to absorb both the first laser light, which is a red light, and the second laser light, which is a near infrared light. That is, the first laser light, which is a red light, is easily absorbed by reduced hemoglobin and is less likely to be absorbed by oxygenated hemoglobin. Further, the second laser light, which is a near infrared light, is less likely to be absorbed by reduced hemoglobin and oxygenated hemoglobin.

Thus, the first laser light is absorbed mainly by reduced hemoglobin and scattered by oxygenated hemoglobin. Accordingly, the intensity of the scattered light of the first laser light received as a biological measurement output by the first light receiver 131 is due to the amount of oxygenated hemoglobin. On the other hand, the second laser light is scattered by the reduced hemoglobin and the oxygenated hemoglobin. Accordingly, the intensity of the scattered light of the second laser light received as a biological measurement output by the second light receiver 132 is due to a total amount of hemoglobin including the reduced hemoglobin and the oxygenated hemoglobin.

The temperature sensor 140 constituting the second measurement unit may be configured by including a thermistor, for example. In this case, the temperature sensor 140 can measure the body temperature of the subject by the thermistor. As a thermistor used as the temperature sensor 140, a thermistor having a known configuration that uses, as a resistor, ceramics whose resistance value varies with temperature can be employed. Thus a detailed description of the thermistor will be omitted. The temperature sensor 140 is not limited to one configured to include a thermistor, and may be configured to include any functional part as long as it can detect temperatures. The temperature sensor 140 constituting the second measurement unit may serve as a temperature sensor configured to measure the body temperature of the subject, for example.

In the measurement apparatus 100, the temperature sensor 140 may be built into a housing of the biosensor 110, or may be separated from the biosensor 110. When the temperature sensor 140 is configured to be separated from the biosensor 110, the temperature sensor 140 may be configured to be connected to the biosensor 110, the controller 150, or the like. The temperature sensor 140 may have various configurations so as to contact a part suitable to measure the temperature (e.g., body temperature) of the subject.

For example, the temperature sensor 140 may be built into a housing of the biosensor 110 illustrated in FIG. 2. In this case, the temperature sensor 140 may be formed into a protrusion protruding from the biosensor 110, for example. The temperature sensor 140 may be connected to the biosensor 110 so as to come in contact with the vicinity of the fingertip of the subject when the measurement apparatus 100 is worn on the subject. Here, the part of the subject measured by the temperature sensor 140 may be a wrist, an arm, an ear, a forehead, a neck, a back, a foot, other parts or any combination thereof, in place of or in addition to a fingertip. Specific aspects in which the temperature sensor 140 comes in contact with the measured part of the subject will be further described in detail below.

The temperature sensor 140 can measure the temperature (e.g., the body temperature) of the subject by coming in contact with the measured part or the vicinity of the measured part of the subject. Further, for example, the temperature sensor 140 may include a temperature detector configured to measure the environmental temperature not coming in contact with the subject, apart from the temperature detector that comes in contact with the measured part or the vicinity of the measured part of the subject. In this manner, the influence of the environmental temperature on the measured body temperature can be taken into consideration by measuring both the body temperature of the subject and the environmental temperature by the temperature sensor 140.

The temperature sensor 140 transmits the information on the measured temperature of the subject to the controller 150. For example, as described above, when the temperature sensor 140 is configured as a thermistor, the resistance value detected by the thermistor may be transmitted to the controller 150. In this case, the controller 150 can calculate the temperature based on the transmitted resistance value. Here, the memory 170 may store the correspondence between the detected resistance value and the temperature value. In this case, the controller 150 can read the temperature value corresponding to the detected resistance value from the memory 170. Further, the memory 170 may store a formula that defines a relationship between the detected resistance value and the temperature. In this case, the controller 150 can calculate the temperature value from the detected resistance value according to the formula stored in the memory 170. In this manner, the controller 150 can measure the temperature (e.g., the body temperature) of a predetermined part of the subject based on the output from the temperature sensor 140. Further, the temperature sensor 140 may be able to measure or calculate the temperature by itself. In this case, the controller 150 may acquire the information of the temperature detected or calculated by the temperature sensor 140.

Referring to FIG. 1 again, the controller 150 includes at least one processor 151 configured to control and manage the entire measurement apparatus 100, including each functional block of the measurement apparatus 100. The controller 150 is configured by including at least one processor 151 such as a Central Processing Unit (CPU) that executes a program that defines a control procedure, and realizes its functions. Such a program is stored, for example, in the memory 170 or an external storage medium connected to the measurement apparatus 100.

According to various embodiments, the at least one processor 151 may be implemented as a single integrated circuit (IC), or a plurality of communicably connected integrated circuits IC and/or discrete circuits. The at least one processor 151 can be executed according to various known technologies.

In one embodiment, the processor 151 includes, for example, one or more circuits or units configured to execute one or more data computing procedures or processes by executing instructions stored in an associated memory. In other embodiments, the processor 151 may be firmware (e.g., a discrete logic component) configured to execute one or more data computing procedures or processes.

According to various embodiments, the processor 151 may include one or more processors, controllers, microprocessors, microcontrollers, application specific integrated circuits (ASICs), digital signal processors, programmable logic devices, field programmable gate arrays, any combination thereof, or any combination of their configurations, and perform the functions of the controller 150 described below.

The controller 150 may calculate values associated with the blood flow amounts based respectively on the output from the first light receiver 131 and from the second light receiver 132 (i.e., photoelectric conversion signals of scattered light). The value based on the output from the first light receiver 131 is referred to as a first value, and the value based on the output from the second light receiver 132 is referred to as a second value. The controller 150 can calculate the first value and the second value by utilizing the Doppler shift.

Here, a measuring method of the first value and the second value by utilizing the Doppler shift by the controller 150 will be described. To measure the first value and the second value, the controller 150 causes the light emitter (i.e., the first laser light source 121 and the second laser light source 122) to emit laser light into the tissue of a living body, and causes the light receivers (i.e., the first light receiver 131 and the second light receiver 132) to receive the light scattered from the tissue of the living body. Then the controller 150 calculates the first value and the second value based on the measurement results of the received laser light.

In the tissue of the living body, the scattered light from the moving blood cells undergoes a frequency shift (a Doppler shift), due to the Doppler effect, that is proportional to the moving speed of the blood cells in the blood. The controller 150 detects a beat signal caused by the light interference between the scattered light from the static tissue and the scattered light from the moving blood cells. The beat signal represents intensity as a function of time. The controller 150 then converts the beat signal into a power spectrum which represents power as a function of frequency. In the power spectrum of the beat signal, the Doppler shift frequency is proportional to the blood cell speed, and the power corresponds to the blood cell amount. Then the controller 150 calculates the first value and the second value by multiplying the power spectrum of the beat signal by the frequency and then integrating the multiplication result.

The controller 150 can calculate the first value PI [ml/min] from P1=K·∫f·P(f) df/(I×I), for example, where K represents a proportionality constant, IxI represents a mean square of the intensity of the received light signal, f represents the frequency, and P(f) represents a power spectrum of the beat signal. The controller 150 may calculate the first value PI from, for example, P1=∫f·P(f) df/(I×I) or P1=f f·P(f) df. That is, the controller 150 may calculate the first value PI by using any one of P1=K·∫f·P(f) df/(I×I), P1=∫f·P(f) df/(I×I), and P1=∫f·P(f) df. The same applies to the second value P2. In other words, the controller 150 may calculate the second value P2 from any one of P2=K·∫f·P(f) df/(I×I), P2=∫f·P(f) df/(I×I), and P2=∫f·P(f) df.

As described above, because the output from the first light receiver 131 is due to the amount of oxygenated hemoglobin in the blood, the first value indicates a value based on the flow rate of the oxygenated hemoglobin. Because the output from the second light receiver 132 is due to the total amount of hemoglobin in the blood, the second value indicates a value based on a flow rate of all hemoglobin in the blood. Because the value calculated based on the flow rate of all hemoglobin in the blood is, in other words, the blood flow amount of the subject, the second value indicates the blood flow amount of the subject. Accordingly, the controller 150 can calculate the blood flow amount of the subject by calculating the second value. In this manner, the measurement apparatus 100 can measure the blood flow amount of the subject.

The controller 150 calculates the SpO₂ of the subject based on the first value and the second value. In this case, the controller 150 can calculate the SpO₂ based on a ratio of the first value to the second value.

Here, the calculation method for SpO₂ by the controller 150 will be described in detail. SpO₂ is calculated from the formula of {HbO₂/(Hb+H HbO₂)}×100, where HbO₂ represents the amount of oxygenated hemoglobin, and Hb represents the amount of reduced hemoglobin (for example, see PTL 1). In this formula, HbO₂ represents the amount of oxygenated hemoglobin, and (Hb+HbO₂) represents a total amount of oxygenated hemoglobin and reduced hemoglobin. Thus, in this embodiment, HbO₂ can correspond to the first value calculated based on the flow rate of oxygenated hemoglobin, and (Hb+HbO₂) can correspond to the second value calculated based on the flow rate of all hemoglobin in the blood. Accordingly, in the above formula, when HbO₂ is replaced with the first value and (Hb+ HbO₂) is replaced with the second value, the index indicating SpO₂ can be calculated from, for example, (first value/second value)×100. In this embodiment, the controller 150 calculates the index indicating SpO₂ from the formula of (first value/second value)×100. In this manner, when the controller 150 calculates the index indicating SpO₂, the measurement apparatus 100 can measure the SpO₂ of the subject. Here, the formula of (first value/second value)×100 is used to calculate the index indicating SpO₂. Therefore, a value acquired from the formula of (first value/second value)×100 may be SpO₂. Further, a value acquired by performing predetermined weighting, e.g., by multiplying a coefficient may be SpO₂. Moreover, a value acquired by using a table for converting the value of (first value/second value) into SpO₂ may be SpO₂.

As described above, the first laser beam, which is red light, is easily absorbed by reduced hemoglobin and less likely to be absorbed by oxygenated hemoglobin. Further, as described above, the second laser beam, which is near-infrared light, is less likely to be absorbed by reduced hemoglobin and oxygenated hemoglobin. Using such properties, in the present disclosure, the controller 150 may calculate SpO₂ based on a difference in absorbance of blood between the first laser light and the second laser light..

The controller 150 may estimate the likelihood that the subject develops altitude sickness (also called altitude impairment) based on the calculated blood flow amount and SpO₂ of the subject. The subject is more likely to develop altitude sickness when SpO₂ decreases or when dehydration occurs. If the subject tends to be dehydrated, insufficient moisture in the blood causes poor blood flow (decrease in the blood flow amount). The controller 150 can estimate the likelihood that the subject develops altitude sickness based on changes in the blood flow amount and SpO₂. The controller 150 may estimate the likelihood of developing altitude sickness by, for example, weighting the blood flow amount and the SpO₂ by using a predetermined algorithm. The measurement apparatus 100 according to this embodiment can measure the SpO₂ and the blood flow amount, and thus is capable of estimating the likelihood of developing altitude sickness based on the two indexes such as the SpO₂ and the blood flow amount. Thus, the measurement apparatus 100 according to this embodiment can estimate the likelihood of developing altitude sickness more accurately compared to a case where the likelihood of developing altitude sickness is estimated based only on SpO₂.

Further, the controller 150 may estimate the health condition of the subject based on the temperature measured by the temperature sensor 140, in addition to the above described blood flow amount and SpO₂ of the subject. Moreover, the controller 150 may estimate cyanosis of the subject, for example, as an estimation of the health condition of the subject.

Cyanosis is a deficiency of oxygen in the blood that causes the skin or mucous membranes to become purplish or blue-violet in color. Cyanosis generally tends to appear on the nail bed and around lips of mouth when oxygen level in the blood is low. Cyanosis is caused by heart disease, pneumonia, pulmonary tuberculosis and the like, and can also be caused by local blood circulation disorders, and is said to particularly strongly appear on the distal portion of the extremities, and lips of mouth and the like. Cyanosis is said to appear in the capillaries of the skin or mucous membranes when the amount of oxygenated hemoglobin (deoxygenated hemoglobin) exceeds 5 g per 100 ml of blood. This is about 66% in terms of oxygen saturation. Cyanosis appears at higher oxygen saturation when red blood cell count increases. Conversely, anemia makes cyanosis less likely to appear even at lower oxygen saturation.

For example, as the symptoms of altitude sickness described above progress, the skin and mucous membranes may become blue-black as a symptom of cyanosis due to a lack of oxygen in the blood. If such a condition becomes severe, emphysema may occur, thus detection in the early stage is desired. Thus, in an embodiment, the controller 150 may estimate the health condition of the subject based on at least any one of the above described blood flow amount, the SpO₂ and the temperature of the subject measured by the temperature sensor 140. Here, as an estimation relating to the health condition of the subject, the controller 150 may estimate the cyanosis of the subject.

As described above, the controller 150 can estimate the likelihood that the subject will have altitude sickness based on the changes in the blood flow amount and the SpO₂. In an embodiment, the controller 150 may estimate the health condition of the subject, for example, cyanosis of the subject, based on the information on the body temperature of the subject. In this case, the controller 150 may estimate the health condition of the subject by performing a predetermined weighting on the measured value of the body temperature of the subject, for example, by using a predetermined algorithm. In the measurement apparatus 100 according to this embodiment, the SpO₂, the blood flow amount and the body temperature can be measured, and thus the health condition, for example, the cyanosis of the subject can be estimated based on these three indexes. Thus, in the measurement apparatus 100 according to this embodiment, the estimation accuracy of the likelihood that the subject will be in a state like cyanosis, for example, is increased, compared to the case where the health condition of the subject is estimated based on any one of the SpO₂, the blood flow amount and the body temperature.

The notification interface 160 notifies information by sounds, vibrations and images. The notification interface 150 may include a speaker, a vibrator, and a display device. The display device may be, for example, a Liquid Crystal Display (LCD), an Organic Electro-Luminescence Display (OELD), an Inorganic Electro-Luminescence Display (IELD), or the like. The notification interface 160 may notify, for example, a measurement result of at least any one of the SpO₂, the blood flow amount and the body temperature of the subject. The notification interface 150 may also notify, for example, information on the likelihood that the subject may be in a state of cyanosis, for example.

The memory 170 can be configured as a semiconductor memory, a magnetic memory, or the like. The memory 170 stores various kinds of information and a program for operating the measurement apparatus 100. The memory 170 may also function as a working memory. The memory 170 may store, for example, the SpO₂ and the blood flow amount of the subject calculated by the controller 140 as the history information. Furthermore, the memory 170 may also store, for example, the temperature of the subject acquired by the controller 150 as the history information.

Further, the memory 170 may also store a correspondence relation between the temperature detected by the temperature sensor 140 and the body temperature of the subject. Specifically, as described above, the memory 170 may store, for example, the correspondence between the resistance value detected by the temperature sensor 140, which is a thermistor, and the temperature value such as the body temperature of the subject. Further, as described above, the memory 170 may store the formula that specifies the relationship between the resistance value detected by the temperature sensor 140, which is a thermistor, and the temperature value such as the body temperature of the subject.

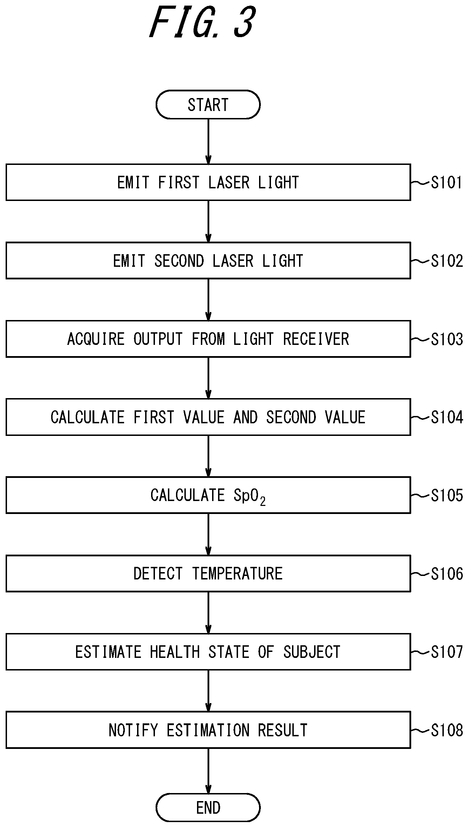

Next, an example of the processing performed by the controller 150 of the measurement apparatus 100 will be described with reference to the flowchart illustrated in FIG. 3. The controller 150 may repeat the flow illustrated in FIG. 3 when the measurement apparatus 100 is activated or when a predetermined input operation is performed for starting the measuring processing. In a case where the controller 150 has functionality which is able to detect whether the measured part is in contact with the measurement unit, the controller 150 may execute the flow illustrated in FIG. 3 when it is determined that the measured part is in contact with the measurement unit.

When the flow operation illustrated in FIG. 3 is started, the controller 150 first causes the first laser light source 121 to emit first laser light (step S101).

The controller 150 causes the second laser light source 122 to emit second laser light (step S102).

When the first laser light is emitted in step S101, the first light receiver 131 receives scattered light from the measured part. When the second laser light is emitted in step S102, the second light receiver 132 receives scattered light from the measured part. The first light receiver 131 and the second light receiver 132 transmit photoelectric conversion signals of the respective scattered lights to the controller 150.

The controller 150 acquires outputs from the first light receiver 131 and the second light receiver 132 (step S103).

The controller 150 calculates the first value based on the output acquired from the first light receiver 131 and the second value based on the output acquired from the second light receiver 132 (step S104). As described above, the first value and the second value calculated in step S104 are values relating to the blood flow amount.

The controller 150 calculates indexes indicated in SpO₂ based on the first value and the second value calculated in step S104, and calculates SpO₂ from the indexes indicated in the SpO₂ (step S105).

The controller 150 detects the temperature (body temperature) of the subject from the temperature sensor 140 (step S106). The controller 150 may cause the temperature sensor 140 to detect the temperature at the timing of step S106. Further, the controller 150 may acquire the information on the temperature constantly detected by the temperature sensor 140 at the timing of step S106.

The controller 150 estimates the health condition of the subject based on at least one of the first value and the second value calculated in step S104, the SpO₂ calculated in step S105, or the temperature detected in step S106 (step S107). Here, at least one of the first value and the second value may be the second value representing the blood flow amount of the subject. Further, in step S107, the controller 150 may estimate the cyanosis of the subject as estimation of the health condition of the subject.

The controller 150 causes the notification interface 160 to notify the information on the result estimated in step S107 (step S108). In step S108, the controller 150 may cause the notification interface 160 to notify the information on the estimation result regarding the cyanosis of the subject, for example.

In this manner, the measurement apparatus 100 according to this embodiment may include the first measurement unit (e.g., the biosensor 110) and the controller 150. Further, the measurement apparatus 100 according to this embodiment may also include the second measurement unit (e.g., the temperature sensor 140). Here, the first measurement unit measures at least one of the percutaneous oxygen saturation (SpO₂) and the blood flow amount of the subject. Further, the second measurement unit measures the body temperature of the subject. The controller 150 also estimates the health condition of the subject based on the information that includes at least one of the measured value of the SpO₂ of the subject and the measured value of the blood flow amount of the subject. Moreover, the controller 150 may estimate the health condition of the subject based on the information that includes the measured value of the body temperature of the subject. Further, the controller 150 may also estimate the cyanosis of the subject, for example, as an estimation of the health condition of the subject.

Further, in this manner, the measurement apparatus 100 may include the notification interface 160. In this case, the notification interface 160 notifies the estimation result by the controller 150.

In this manner, the measurement apparatus 100 can estimate the health condition such as the cyanosis of the subject by putting multiple detection results together. Therefore, according to the measurement apparatus 100, compared with the case where the health condition of the subject is estimated based on each detection result, estimation accuracy will be improved. In this manner, according to the measurement apparatus 100, usability will be improved.

Further, according to the measurement apparatus 100, the blood flow amount, the SpO₂ and the body temperature can be measured by one apparatus. Thus, according to the measurement apparatus 100, there is no need to measure the blood flow amount, the SpO₂ and the body temperature with separate devices, and thus the convenience can be enhanced when the health condition of the subject is estimated.

In the above described steps S104 and S105, the controller 150 calculates the first value and the second value as a value relating to the blood flow amount of the subject, and calculates the SpO₂ based on these values. However, the values calculated by the controller 150 are not limited to the above described values, and may be various kinds of values.

For example, the controller 150 can calculate the pulse rate of the subject based not only on the calculation of the SpO₂ but also on the change in the reception light intensity over time in the first light receiver 131 and/or the second light receiver 132. Specifically, the controller 150 can calculate the period of the reception light intensity from the change in the reception light intensity over time, and can calculate the pulse rate per unit time based on the period. Moreover, the controller 150 can calculate the Perfusion Index (PI) value of the subject based on the change in the reception light intensity over time in the first light receiver 131 and/or the second light receiver 132. PI, also known as the perfusion index, is expressed as the ratio of the pulsation component to non-pulsation component in the blood flow. The controller 150 can calculate the PI by calculating the ratio of the pulsation component to the non-pulsation component in the blood flow from the change in the reception light intensity over time. Moreover, the controller 150 can calculate the respiratory rate of the subject based on the change in the reception light intensity over time in the first light receiver 131 and/or the second light receiver 132. For example, the controller 150 may calculate the respiratory rate by extracting the low frequency component of the change in the reception light intensity over time in the first light receiver 131 and/or the second light receiver 132.

In this manner, in an embodiment, the controller 150 may calculate the above described each value. Further, in this manner, in an embodiment, the controller 150 may estimate the health condition of the subject in consideration of the calculated each value described above. That is, the controller 150 may calculate the respiratory rate of the subject based at least one of the measured value of the SpO₂ of the subject and the measured value of the blood flow amount of the subject, for example. In this case, the controller 150 may estimate the health condition of the subject based on not only the above information but also the information including the respiratory rate of the subject. Further, the controller 150 may calculate the Perfusion Index (PI) of the subject based on at least one of the measured value of the SpO₂ of the subject and the measured value of the blood flow amount of the subject, for example. In this case, the controller 150 may estimate the health condition of the subject based on not only the above described information but also the information including the PI of the subject.

Further, if at least one of the above described values falls below each corresponding predetermined value, the controller 150 may control so that the notification interface will notify an estimation regarding the health condition of the subject. Here, the above described values may be a measured value of the SpO₂ of the subject, a measured value of the blood flow amount, a measured value of the body temperature, a calculated value of the respiratory rate and a calculated value of the PI. A predetermined value corresponding to each value may be set in advance to determine that the health condition of the subject is not normal, abnormal or declining if these values fall more than a certain degree. In this manner, a predetermined value set corresponding to each value may be stored in the memory 170, for example.

According to the above described embodiment, estimation regarding the health condition such as the cyanosis of the subject can be made by putting more detection results together. Therefore, according to such an embodiment, accuracy of estimation regarding the health condition of the subject can be further improved. Therefore, according to such an embodiment, the usability is further improved.

### (Second Embodiment)

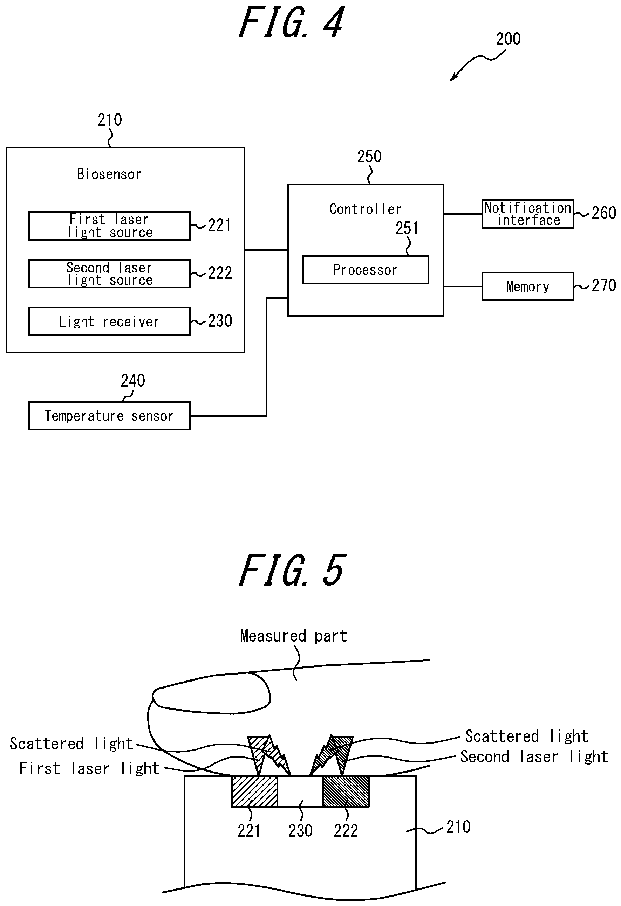

FIG. 4 is a functional block diagram illustrating a schematic configuration of a measurement apparatus 200 according to a second embodiment. The measurement apparatus 200 according to this embodiment includes a biosensor 210, a temperature sensor 240, a controller 250, a notification interface 260 and a memory 270.

In the measurement apparatus 100 according to the first embodiment, the biosensor 110 includes two light receivers such as the first light receiver 131 and the second light receiver 132. On the other hand, the measurement apparatus 200 according to the second embodiment is different from the measurement apparatus 100 according to the first embodiment in that the biosensor 210 includes only one light receiver 230.

That is, in this embodiment, the biosensor 210 includes two light emitters such as the first laser light source 221 and the second laser light source 222, and a light receiver 230. The functions of the first laser light source 221 and the second laser light source 222 are the same as those of the first laser light source 121 and the second laser light source 122 in the first embodiment, respectively. That is, the first laser light source 221 emits the first laser light and the second laser light source 222 emits the second laser light. The first laser light source 221 and the second laser light source 222 emit the first laser light and the second laser light, respectively, at different timings. The first laser light source 221 and the second laser light source 222 alternately output laser light, for example. That is, in the measurement process by the measurement apparatus 200, the first laser light and the second laser light are alternately emitted to the measured part, for example, at predetermined time intervals.

The light receiver 230 is configured as, for example, a so-called multi-frequency-responsive PD capable of detecting scattered lights of both of the first laser light (red light) and the second laser light (near infrared light). Thus, the second light receiver 232 detects the scattered light of the first laser light when the first laser light is emitted to the measured part, and detects the scattered light of the second laser light when the second laser light is emitted to the measured part. The biosensor 210 transmits a photoelectric conversion signal of the scattered light received by the light receiver 230 to the controller 250.

FIG. 5 is a schematic diagram for illustrating an example of a usage state of the biosensor 210 in the measurement apparatus 200. As schematically illustrated in FIG. 5, the light receiver 230 receives, from the measured part, scattered light of the first laser light emitted by the first laser light source 221 and scattered light of the second laser light emitted by the second laser light source 222. Since the first laser light and the second laser light are alternately emitted as described above, the light receiver 230 alternately receives the scattered first laser light and scattered second laser light. Therefore, although FIG. 5 illustrates the first laser light, the second laser light and scattered lights of the first laser light and the second laser light, in reality either the first laser light or the second laser light is emitted to the measured part at a certain point in time, and the light receiver receives the scattered light of the laser light being emitted. The light receiver 230 is disposed at a position of the biosensor 210 where the scattered lights of the laser lights emitted by the first laser light source 221 and the second laser light source 222 can be received.

Referring to FIG. 4 again, since the function of the temperature sensor 240 is the same as that of the temperature sensor 140 according to the first embodiment, the detailed description will be omitted here. Further, the controller 250 includes at least one processor 251 configured to control and manage the entire measurement apparatus 200, including each functional block thereof. Functions of the controller 250 and the processor 251 are the same as those of the controller 150 and the processor 151, respectively, of the first embodiment. Thus, detailed descriptions will be omitted here. Further, functions of the notification interface 260 and the memory 270 are the same as those of the notification interface 160 and the memory 170, respectively, of the first embodiment. Thus, detailed descriptions will be omitted here.

In the measurement apparatus 200 according to this embodiment, the controller 250 measures the blood flow amount, the SpO₂ and the temperature according to the flow similar to the one described with reference to FIG. 3, and estimates the health condition such as cyanosis, for example, of the subject. In this embodiment, the controller 250 acquires the output from the light receiver 230 in step S103. The controller 240 calculates the first value or the second value in step S104, depending on whether the output acquired from the light receiver 230 is the scattered light of the first laser light or of the second laser.

As described above, the measurement apparatus 200 according to this embodiment also emits laser light to the measured part and measures SpO₂. Thus, the measurement apparatus 200 can more accurately measure the SpO₂ than an apparatus that uses, for example, light of a wide wavelength band. Further, the measurement apparatus 200 can also estimate the health condition such as cyanosis of the subject by putting multiple detection results together. In this manner, according to the measurement apparatus 200, not only usability but also convenience when the health condition of the subject is estimated can be improved. Moreover, the measurement apparatus 200 according to this embodiment can receive the scattered light of the first laser light and the scattered light of the second laser light by using one light receiver 230 that corresponds to multiple frequencies. Thus, the biosensor 210 and the measurement apparatus 200 can be miniaturized, compared to a case where the scattered light of the first laser light and the scattered light of the second laser light are received by two separate light receivers, respectively.

### (Third embodiment)

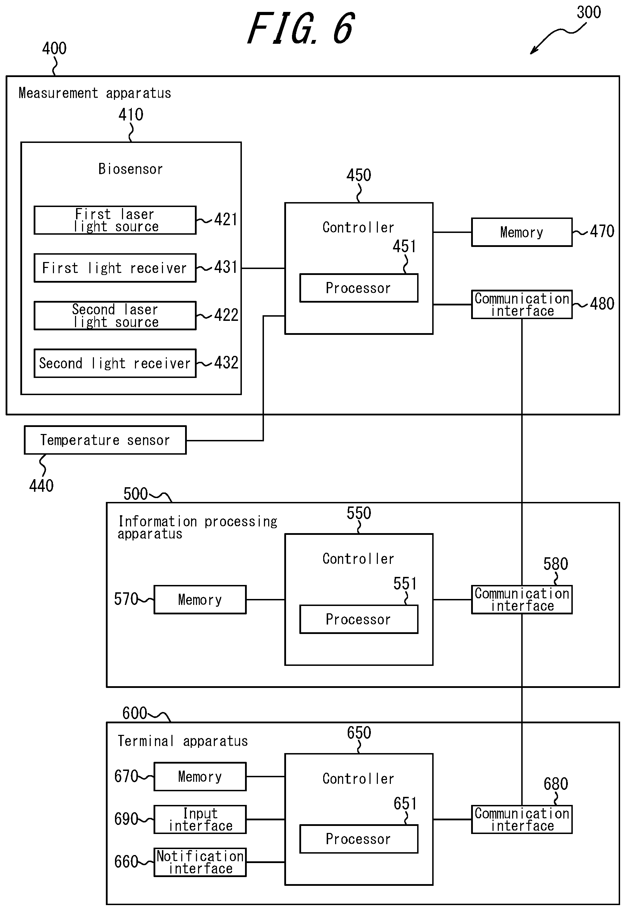

FIG. 6 is a functional block diagram illustrating a schematic configuration of a measurement system 300 according to a third embodiment. The measurement system 300 includes a measurement apparatus 400, an information processing apparatus 500 and a terminal apparatus 600. The information processing apparatus 500 is communicably connected to the measurement apparatus 400 and the terminal apparatus 600 via wired communication, wireless communication, or a combination thereof. Further, the measurement apparatus 400 and the terminal apparatus 600 may directly communicate with each other. Further, the network connecting the measurement apparatus 400, the information processing apparatus 500 and the terminal apparatus 600 may be the Internet, a wireless LAN, or the like.

The measurement apparatus 400 is an apparatus configured to measure a biological measurement output by emitting laser light to the measured part. The measurement apparatus 400 may transmit the information on the measured biological measurement output to the information processing apparatus 500.

The information processing apparatus 500 may be configured as, for example, a server apparatus such as a computer. The information processing apparatus 500 may calculate the blood flow amount and the SpO₂ of the subject based on the information on the biological measurement output acquired from the measurement apparatus 400. Further, the information processing apparatus 500 may acquire the temperature detected by the temperature sensor 440 in the measurement apparatus 400. The information processing apparatus 500 may estimate the health condition such as cyanosis of the subject. The information processing apparatus 500 may store the calculation results of the blood flow amount and the SpO₂, the detected temperature and the information on the estimated health condition of the subject. The information processing apparatus 500 may transmit the calculation results of the blood flow amount and the SpO₂, the detected temperature and the information on the estimated health condition of the subject to the terminal apparatus 600.

The terminal apparatus 600 may be configured as, for example, a personal computer, a smart phone, a tablet computer, or the like. The terminal apparatus 600 may be owned by the subject. The terminal apparatus 600 may notify, based on the calculation results of the blood flow amount and the SpO₂, the detected temperature, and the information on the estimated health condition of the subject, acquired from the information processing apparatus 500.

The measurement apparatus 400 includes the biosensor 410, the temperature sensor 440, the controller 450, the memory 470 and the communication interface 480. The biosensor 410 includes the first laser light source 421, the second laser light source 422, the first light receiver 431 and the second light receiver 432. Functions of the first laser light source 421, the second laser light source 422, the first light receiver 431 and the second light receiver 432 are the same as those of the first laser light source 121, the second laser light source 122, the first light receiver 131 and the second light receiver 132, respectively, of the first embodiment. Further, the function of the temperature sensor 440 is the same as that of the temperature sensor 140 according to the first embodiment. As with the measurement apparatus 100 according to the first embodiment, the measurement apparatus 400 according to this embodiment can acquire the biological measurement output.

The controller 450 includes at least one processor 451 configured to control and manage the entire measurement apparatus 400, including each functional block thereof. The controller 450 includes at least one processor 451 such as a CPU configured to execute a program defining a control procedure and thus realizes its functions. Such a program is stored in, for example, the memory 470 or an external storage medium connected to the measurement apparatus 400. The processor 451 may have a configuration similar to, for example, that of the processor 151 according to the first embodiment. Thus, detailed descriptions will be omitted here. The controller 450 controls acquisition of the biological measurement output by the biosensor 410 and transmits the acquired information on the biological measurement output to the information processing apparatus 500 via the communication interface 480.

The memory 470 may be configured as a semiconductor memory, a magnetic memory, or the like. The memory 470 stores various kinds of information and/or a program for operating the measurement apparatus 400. The memory 470 may also function as a working memory. The memory 470 may store, for example, data such as the information on the biological measurement output (i.e., received light intensities of scattered light and/or temperatures) acquired by the biosensor 410.

The communication interface 480 transmits and receives various kinds of information by performing wired communication, wireless communication, or a combination thereof, with the information processing apparatus 500. For example, the communication interface 580 transmits the information on the biological measurement output measured by the measurement apparatus 400 to the information processing apparatus 500.

The information processing apparatus 500 includes a controller 550, a memory 570 and a communication interface 580.

The controller 550 includes at least one processor 551 configured to control and manage the entire information processing apparatus 500, including each functional block thereof. The controller 550 includes at least one processor 551 such as a CPU configured to execute a program defining a control procedure and thus realizes its functions. Such a program is stored in, for example, the memory 570 or an external storage medium connected to the information processing apparatus 500. The processor 541 may have a configuration similar to, for example, the configuration of the processor 151 illustrated in the first embodiment. Thus, detailed descriptions will be omitted here. The controller 550 may calculate the blood flow amount and the SpO₂ of the subject based on the information on the biological measurement output acquired from the measurement apparatus 400. The controller 550 may estimate the health condition of the subject based on at least one of the acquired blood flow amount and SpO₂, and the temperature (body temperature). The calculation method of the blood flow amount and the SpO₂, the method of acquiring the temperature, and the estimation method of the health condition are similar to those described in the first embodiment. Thus, detailed descriptions will be omitted here.

The memory 570 may be configured as a semiconductor memory, a magnetic memory, or the like. The memory 560 stores various kinds of information, programs for operating the information processing apparatus 500, and the like. The memory 570 may also function as a working memory. The memory 560 may store, for example, the information on the biological measurement output acquired from the measurement apparatus 400. The memory 570 may store various kinds of information used for calculation of the blood flow amount and the SpO₂, acquisition of the temperature and estimation regarding the health condition by the controller 550.

The communication interface 580 transmits and receives various kinds of information through wired communication, wireless communication, or a combination thereof, with the measurement apparatus 400 and the terminal apparatus 600. For example, the communication interface 570 receives the information on the biological measurement output from the measurement apparatus 400. Further, for example, the communication interface 580 transmits the blood flow amount and the SpO₂ calculated by the information processing apparatus 500, and the temperature and the information on estimation regarding the health condition acquired from the information processing apparatus 500 to the terminal apparatus 600.

The terminal apparatus 600 includes a controller 650, a notification interface 660, a memory 670, a communication interface 680 and an input interface 690.

The controller 650 includes at least one processor 651 configured to control and manage the entire terminal apparatus 600, including each functional block thereof. The controller 650 includes at least one processor 651 such as a CPU configured to execute a program defining a control procedure and thus realizes its functions. Such a program is stored in, for example, a memory 670 or an external storage medium connected to the terminal apparatus 600. The processor 651 may have a configuration similar to that of the processor 151 illustrated in the first embodiment, for example. Thus, detailed descriptions will be omitted here. The controller 650 may cause the notification interface 650 to notify the blood flow amount and the SpO₂, the temperature and the information on estimation regarding the health condition of the subject acquired from the information processing apparatus 500.

The notification interface 660 notifies the information through sounds, vibrations, images, or the like. The functions and the configuration of the notification interface 660 may be the same as those of the notification interface 160 described in the first embodiment. Thus, detailed descriptions will be omitted here.

The memory 670 may be configured as a semiconductor memory, a magnetic memory, or the like. The memory 670 stores various kinds of information, programs for operating the terminal apparatus 600, and the like. The memory 670 may also function as a working memory. The memory 670 may store, for example, the blood flow amount and the SpO₂, the temperature and the information on estimation regarding the health condition acquired from the information processing apparatus 500.

The communication interface 680 transmits and receives various kinds of information through wired communication, wireless communication, or a combination thereof with the information processing apparatus 500. For example, the communication interface 680 receives the blood flow amount and the SpO₂, the temperature, and the information on estimation regarding the health condition from the information processing apparatus 500.

The input interface 690 is configured to receive an input operation from a user (e.g., a subject) of the terminal apparatus 600 and configured as, for example, an operation button (an operation key). The input interface 680 may be configured as a touch panel that is configured to display an operation key for receiving an input operation from the user in a portion of the display device and may receive a touch input operation by the user.

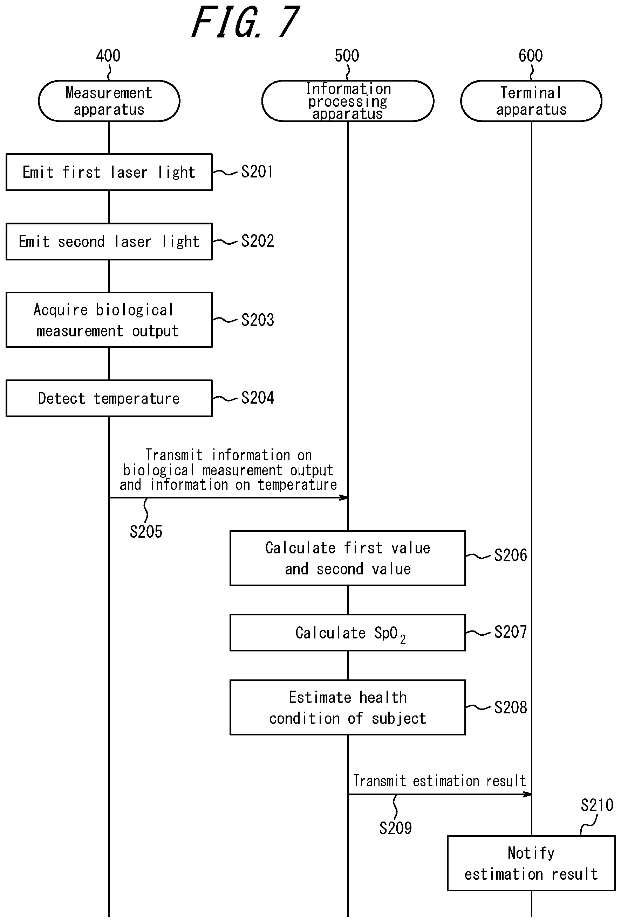

FIG. 7 is a sequence diagram illustrating an example of a control procedure by the measurement system 300. The process illustrated in FIG. 7 is executed when, for example, the measurement apparatus 400 is activated or a predetermined input operation for starting the measuring process is performed. In a case where the controller 450 of the measurement apparatus 400 has functionality which is able to detect whether the measured part is in contact with the measurement unit, the process illustrated in FIG. 7 may be executed when it is determined that the measured part is in contact with the measurement unit.

The measurement apparatus 400 causes the first laser light source 421 to emit the first laser light (step S201).

The measurement apparatus 400 causes the second laser light source 422 to emit the second laser light (step S202).

The measurement apparatus 400 acquires the biological measurement output from the first light receiver 431 and the second light receiver 432 (step S203).

The measurement apparatus 400 detects the temperature (body temperature) of the subject from the temperature sensor 440 (step S204).

The measurement apparatus 400 transmits the information on the acquired biological measurement output and the detected temperature information to the information processing apparatus 500 via the communication interface 480 (step S205).

Upon receiving the information on the biological measurement output and the temperature information from the measurement apparatus 400, the information processing apparatus 500 calculates the first value and the second value based on the biological measurement outputs (step S206).

The information processing apparatus 500 calculates the SpO₂ based on the first value and the second value calculated in step S206 (step S207).

The information processing apparatus 500 estimates the health condition of the subject based on at least one of the blood flow amount (i.e., the second value), the SpO₂ and the temperature (step S208).

The information processing apparatus 500 transmits the blood flow amount, the SpO₂, the temperature and the information on the health condition to the terminal apparatus 600 via the communication interface 580 (step S209).

Upon receiving the blood flow amount and the SpO₂, the temperature and the information on estimation regarding the health condition from the information processing apparatus 500, the terminal apparatus 600 causes the notification interface 660 to notify the information acquired (step S210).

In this embodiment, the biosensor 410 of the measurement apparatus 400 has been described as having a configuration similar to that of the biosensor 110 of the first embodiment. However, the biosensor 410 may have a configuration similar to that of the biosensor 210 of the second embodiment.

In this embodiment, the information processing apparatus 500 has been described as calculating the blood flow amount and the SpO₂ and acquiring the temperature to estimate the health condition. However, for example, the measurement apparatus 200 may perform calculation processing of the blood flow amount and the SpO₂, acquisition processing of the temperature, and estimation processing regarding the health condition. In this case, the measurement apparatus 400 may transmit the calculation results of the blood flow amount and the SpO₂, the acquisition results of the temperature and the estimation results regarding the health condition to the information processing apparatus 500. Further, the measurement system 300 may not include the information processing apparatus 500. In this case, the measurement apparatus 400 may transmit the calculation results of the blood flow amount and the SpO₂, the detected temperature and the estimation results regarding the health condition to the terminal apparatus 600.

In this manner, in the measurement system 300 according to this embodiment, the laser light is emitted to the measured part to calculate the SpO₂, thus measurement accurately of the SpO₂ is improved compared to a case where light of wide wavelength band is used, for example. Further, the measurement system 300 can estimate the health condition such as cyanosis of the subject by putting multiple detection results together. In this manner, according to the measurement system 300, not only usability but also convenience for estimating the health condition of the subject can be improved.

Some embodiments have been described in order to provide a complete and clear disclosure. However, the appended claims are not limited to the above embodiments, and should be constructed so as to encompass all modifications and alternative configurations that can be created by those skilled in the art within the scope of the fundamentals described in this description. Further, each element illustrated in some embodiments may be combined in any appropriate manner.

The measurement apparatuses (the measurement apparatuses 100, 200, and 400) described in the above embodiments can be mounted in various devices.

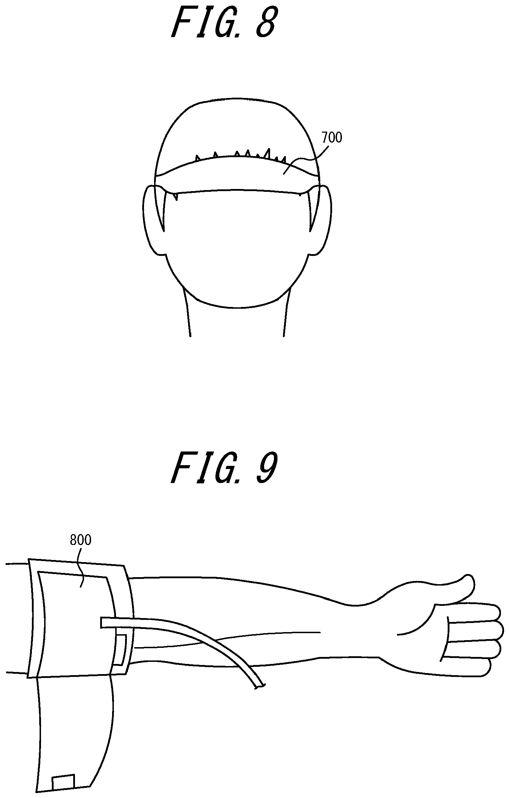

For example, the measurement apparatus 100, 200, or 400 may be mounted in a cerebral blood flow meter configured to measure the cerebral blood flow. The cerebral blood flow meter is a device configured to measure the cerebral blood flow by emitting laser light to the brain. As illustrated in FIG. 8, for example, a subject uses a cerebral blood flow meter 700 by wrapping a measurement member having a strip-like shape about his/her head. The measurement apparatus 100, 200, or 400 may be mounted in the measurement member. When the measurement apparatus 100, 200, or 400 is mounted in the cerebral blood flow meter 700, the subject can activate the cerebral blood flow meter 700 and the measurement apparatus 100, 200, or 400 in a state in which the measurement member of the cerebral blood flow meter 700 is wrapped about his/her head. Thus, the subject can measure the cerebral blood flow, the blood flow amount, and the SpO₂ simultaneously. In this case, the temperature sensor 140, 240 or 440 may be built into the cerebral blood flow amount meter 700 or provided separately from the cerebral blood flow amount meter 700 and connected to the cerebral blood flow amount meter 700. Further, in this case, the cerebral blood flow meter 700 can estimate the health condition of the subject based on at least one of the measured cerebral blood flow, the blood flow amount, the SpO₂, and the detected temperature. Thus, the estimation accuracy is enhanced compared to a case where the health condition of the subject is estimated based only on the SpO₂, for example.

For example, the measurement apparatus 100, 200, or 400 may be mounted in a blood-pressure gauge configured to measure a blood pressure. A blood-pressure gauge may be, for example, a known upper-arm type blood-pressure gauge configured to measure a blood pressure at an upper arm by using a cuff (an arm band). As illustrated in FIG. 9, for example, a subject uses a blood-pressure gauge 800 by wrapping a cuff about his/her upper arm. The measurement apparatus 100, 200, or 400 may be mounted in the cuff. In this case, the temperature sensor 140, 240 or 440 may be built into the cuff of the blood-pressure gauge 800 or may be provided separately from the cuff of the blood-pressure gauge 800 and connected to the blood-pressure gauge 800. In a case where the measurement apparatus 100, 200, or 400 is mounted in the blood-pressure gauge 800, the subject can activate the blood-pressure gauge 800 with the cuff wrapped about his/her upper arm, and the measurement apparatus 100, 200, or 400. In this manner, the subject can measure the blood pressure, the blood flow amount and the SpO₂, and detect the temperature simultaneously. In this case, the blood-pressure gauge 800 can estimate the health condition of the subject based on at least one of the measured blood pressure, the blood flow amount and the SpO₂ and the detected temperature. Thus, the estimation accuracy is enhanced compared to a case where the health condition is estimated based only on SpO₂.

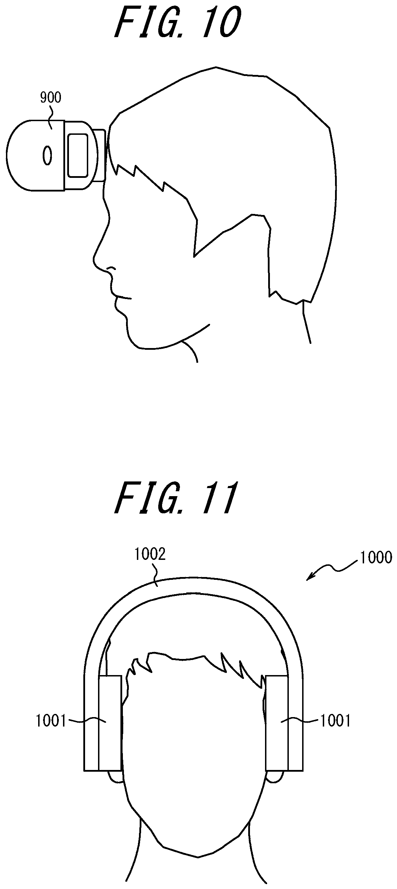

For example, the measurement apparatus 100, 200, or 400 may be mounted in a thermometer configured to measure the body temperature. As illustrated in FIG. 10, for example, a thermometer 900 is brought into contact with human skin to measure the skin temperature. In this case, the thermometer 900 may measure the body temperature of the subject by the temperature sensor 140, 240 or 440. When the measurement apparatus 100, 200, or 400 is mounted in the thermometer 900, the subject can activate the measurement apparatus 100, 200, or 400 when bringing the thermometer 900 into contact with the skin to measure the body temperature. In this manner, the subject can measure the body temperature, the blood flow amount, and the SpO₂ simultaneously. In this case, the thermometer 900 can estimate the health condition of the subject based on the measured body temperature, the blood flow amount, and the SpO₂. Thus, the estimation accuracy is enhanced compared to a case where the health condition of the subject is estimated based only on SpO₂.

The measurement apparatus 100, 200, or 400 may be mounted in various kinds of apparatus capable of measuring the information on a living body, other than the cerebral blood flow meter 700, the blood-pressure gauge 800, and the thermometer 900.

Further, the controller of each of the embodiments has been described as estimating the health condition of the subject based on at least one of the blood flow amount, the SpO₂ and the temperature. However, the controller of each of the embodiments may detect the blood pressure, the dehydration state, the relaxed state, the autonomic state, or other symptoms such as the heart disease, based on at least one of the blood flow amount, the SpO₂ and the temperature. Further, in this case, the controller may detect based on the calculated value of respiratory rate and the calculated value of PI, in addition to at least one of the blood flow amount, the SpO₂ and the temperature.

In the above embodiment, the measured part detected by the biosensor 110, 210 and 410 and/or the measured part detected by the temperature sensor 140, 240 and 440 has/have been described mainly as a finger. However, in the above embodiment, the measured part does not necessary have to be a finger. For example, as illustrated in FIG. 8, the measured part may be the forehead, the side of the head or the temple of the subject or the areas near them. Further, as illustrated in FIG. 9, for example, the measured part may be the upper arm of the subject or the area near the upper arm. Moreover, for example, as illustrated in FIG. 10, the measured part may be the forehead of the subject or the area near the forehead. The measured part may be, for example, the wrist, the arm, the ear, the forehead, the neck, the back, the foot, other parts, or any combination thereof, as described above.

Here, a specific configuration of the measurement apparatus when the measured part is a temple or the vicinity of the temple will be described. FIG. 11 is a diagram schematically illustrating an example where a measuring instrument 1000 including a measurement apparatus configured to measure the temple or the vicinity of the temple is worn. The measuring instrument 1000 includes two holders 1001 and a head band 1002 configured to couple the two holders 1001.

In a state where the measuring instrument 1000 is worn, the two holders 1001 come into contact with the left and right temples or the vicinities thereof, respectively, of the subject and maintain the wearing state. The holders 1001 may be shaped so as not to cover the subject's ears when the measuring instrument 1000 is worn. For example, the holders 1001 may be configured to come in contact with the temples above the ears. In this case, the subject's ears are not covered when the subject wears the measuring instrument 1000, thus the subject can hear the ambient sounds. Accordingly, the safety of the subject can be easily ensured as compared to a case where the subject's ears are covered.

The head band 1002 may have, for example, an arch shape as illustrated in FIG. 11. The measuring instrument 1000 is worn by the subject in such a manner that, for example, the head band 1002 locates on top of the head. The head band 1002 may be designed such that, for example, the length or the curvature thereof is adjustable to the subject's head. The head band 1002 may be made from a member having rigidity such as stainless steel or carbon fiber. In the state where the measuring instrument 1000 is worn, the head band 1002 may maintain the wearing state by pressing the two holders 1001 against the subject's body.

At least one of the holders 1001 includes a measurement apparatus. The measurement apparatus included in the holder 1001 may be, for example, any one of the measurement apparatuses of the first to third embodiments described above. In the following description, the holder 1001 will be described as including the measurement apparatus 200 described in the second embodiment.

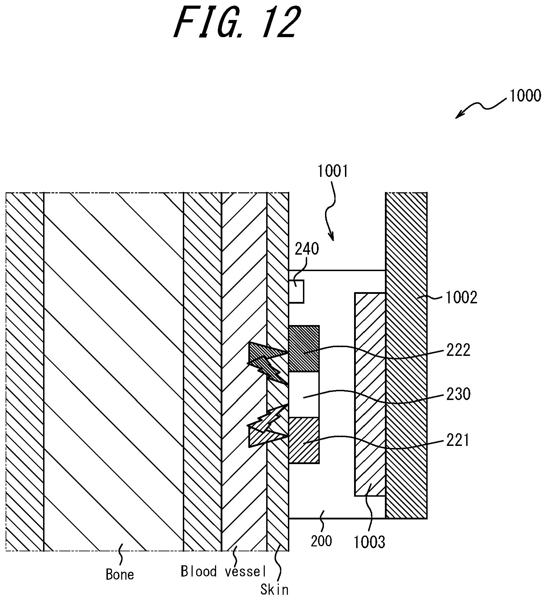

FIG. 12 is a partial cross-sectional view of the measuring instrument 1000 illustrated in FIG. 11 and schematically illustrates the holders each having the measurement apparatus 200. As illustrated in FIG. 12, each of the holders 1001 includes the measurement apparatus 200. The measurement apparatus 200 includes the first laser light source 221, the second laser light source 222, and the light receiver 230 as described in the second embodiment. In the state where the measuring instrument 1000 is worn, the laser light (measuring light) emitted by the first laser light source 221 and the second laser light source 222 is irradiated on the superficial temporal artery. The light receiver 230 receives the scattered light of the measuring light at the superficial temporal artery. That is, the measuring instrument 1000 calculates the blood flow amount and the SpO₂ by using the scattered light at the superficial temporal artery. The blood vessels in the superficial temporal artery are larger than, for example, those in the fingertip, and thus facilitate acquisition of the biological information. Further, the blood vessels in the superficial temporal artery are larger than, for example, those in the fingertip, which allows the blood flow to be stable. Thus, the blood flow amount and the SpO₂ can be more accurately measured by irradiating the measuring light to the superficial temporal artery to acquire the biological information. Further, as illustrated in FIG. 12, the temperature sensor 240 comes in contact with the temple of the subject and detects the temperature (body temperature) of the subject. As an alternative example of the example illustrated in FIG. 12, the temperature sensor 240 is not built into the measurement apparatus 200, and may be disposed outside the measurement apparatus 200 and connected to the measurement apparatus 200, and the like.

As illustrated in FIG. 12, the measurement apparatus 200 may be connected to the head band 1002 via a connection 1003. The connection 1003 functions as a buffer for reducing vibration transmitted from the head band 1002 to the measurement apparatus 200. The connection 1003 functions as, for example, a reducing portion configured to reduce vibration transmitted from the head band 1002 to the measurement apparatus 200. The connection 1003 may function as a damper, for example. The connection 1003 may be made of a resilient material capable of reducing vibration. The connection 1003 may be made of spring, rubber, silicone resin, gel, fabric, sponge, paper, other members, or any combination thereof. The connection 1003 may be, for example, a fluid-filled damper containing a fluid (i.e. liquid or gas). The fluid may be a viscous liquid. The connection 1003 makes it difficult for the vibration of the head band 1002 to be transmitted to the measurement apparatus 200. Thus, the position of the measurement apparatus 200 with respect to the measured part is less likely to change. In this manner, the measurement apparatus 200 can measure the blood flow amount and the SpO₂ more accurately. Further, the measurement apparatus 200 can detect the temperature more accurately.

In the measuring instrument 1000, the measurement apparatus included in the holder 1001 is not limited to the measurement apparatus 200 described in the second embodiment and may be, for example, the measurement apparatus 100 described in the first embodiment. Further, one of the holders 1001 may include the first laser light source 121 and the first light receiver 131 described in the first embodiment, and the other one of the holders 1001 may include the second laser light source 122 and the second light receiver 132 described in the first embodiment. Further, in the measuring instrument 1000, both of the holders 1001 may include the temperature sensor 240 or one of the holders 1001 may include the temperature sensor 240. When each of both holders 1001 includes the temperature sensor 240, for example, the average of the temperatures detected by two temperature sensors 240 may be regarded as the body temperature of the subject.

In the above embodiments, it has been described that the biosensor emits laser light from two laser light sources such as the first laser light source and the second laser light source. However, one of the first laser light source and the second laser light source may be configured as a light source other than the laser light source, such as a Light Emitting Diode (LED). When an LED light source is used in place of the first laser light source, the LED light source emits red light. When the LED light source is used in place of the second laser light source, the LED light source emits near infrared light. When the LED light source is used in place of the laser light source, the controller calculates the first value PI and the second value P2 based on, for example, an intensity of light received by the light receiver with respect to the amount of light emitted by the LED light source. For example, when the LED light source is used in place of the first laser light source, the controller calculates the first value PI based on the intensity of the light received by the first light receiver with respect to the amount of the light emitted by the LED light source. A relationship between a ratio of the received amount of the light to the amount of emitted light and the first value PI may be stored in advance as a table, for example, in the memory. The controller can calculate the first value PI with reference to the table.

Next, another specific configuration of the measurement apparatus in a case where the measured part is a temple or a vicinity of the temple will be described.

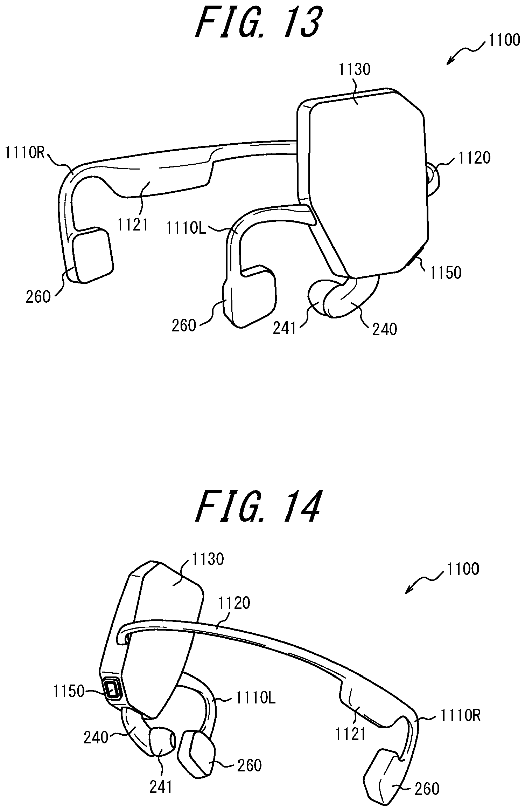

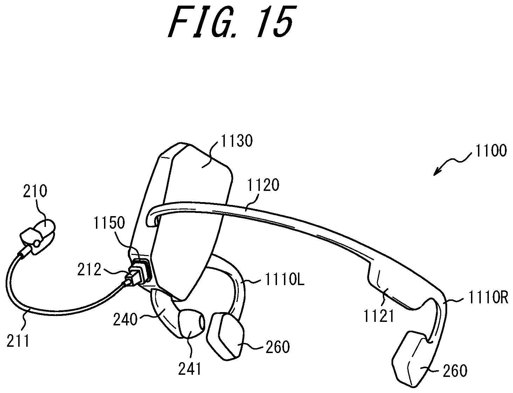

FIG. 13 is a schematic external perspective view of the measuring instrument 1100 according to an embodiment. FIG. 14 is an external perspective view of the measuring instrument in FIG. 13 viewed from a different direction. That is, each of FIG. 13 and FIG. 14 illustrates an external perspective view of the same measuring instrument 1100 when viewed from different directions. Further, FIG. 15 is a schematic external perspective view of the measuring instrument 1100 to which the biosensor 210 is connected.

The measuring instrument 1100 is used while being worn by the subject. In this embodiment, the measuring instrument 1100 is worn on the head of the subject. The measuring instrument 1100 according to this embodiment is worn, in particular, on the auricles of the subject. The measuring instrument 1100 measures the biological information of the subject while being worn on the auricles of the subject.

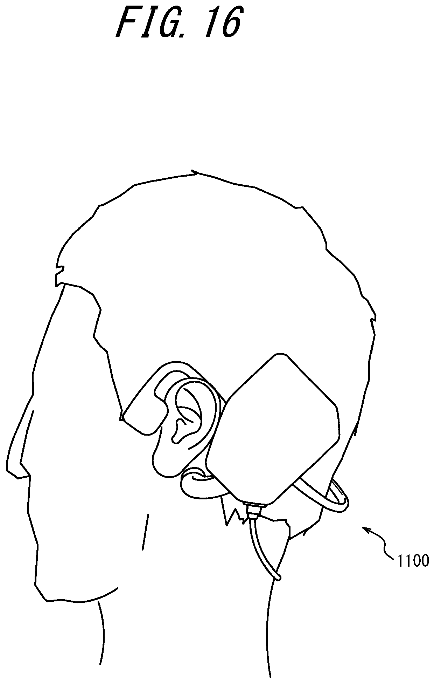

The measuring instrument 1100 includes a first wearing portion 1110R, a second wearing portion 1110L and a coupling portion 1120. The first wearing portion 1110R is worn on the right auricle of the subject. That is, when the measuring instrument 1100 is worn, the first wearing portion 1110R is in contact with the base on the parietal side of the right auricle of the subject and maintains the wearing state of the measuring instrument 1100. The second wearing portion 1110L is worn on the left auricle of the subject. That is, when the measuring instrument 1100 is worn, the second wearing portion 1110L is in contact with the base on the parietal side of the left auricle of the subject. For example, as illustrated in FIG. 16, in the state where the subject wears the measuring instrument 1100, the measuring instrument 1100 is supported by the first wearing portion 1110R and the second wearing portion 1110L worn on each auricle.

The first wearing portion 1110R and the second wearing portion 1110L may have a curved shape illustrated in FIGS. 13 to 15 as an example so as to be easily supported by the right auricle and the left auricle, respectively, when the measuring instrument 1100 is worn. The first wearing portion 1110R and the second wearing portion 1110L may have symmetrical shapes. Hereinafter, when the first wearing portion 1110R and the second wearing portion 1110L are not distinguished, they are collectively referred to as a wearing portion 1110.

The coupling portion 1120 couples the first wearing portion 1110R and the second wearing portion 1110L. The coupling portion 1120 has a curved shape and is configured to be located on the occipital side of the subject when the measuring instrument 1100 is worn. The first wearing portion 1110R, the second wearing portion 1110L and the coupling portion 1120 may be configured symmetrically.

The coupling portion 1120 may be formed in a shape that does not interfere with the attachment of other devices to the head. For example, the subject may wear a helmet, glasses, a hat and the like as other equipment. The coupling portion 1120 may be formed to allow the subject to wear a helmet, glasses or a hat even if the subject wears the measuring instrument 1100. For example, the coupling portion 1120 may be formed so as to be disposed on the neck side of the occipital of the subject when the measuring instrument 1100 is worn. The coupling portion 1120 may be formed so as to cover the top of the head.

The coupling portion 1120 is provided with a body 1130 that has a substrate (e.g., a substrate including the controller 250 and the like) configured to control the measurement processing by the measuring instrument 1100. That is, the body 1130 is coupled to the wearing portion 1110 via the coupling portion 1120, and is supported by the wearing portion 1110 when the measuring instrument 1100 is worn. The body 1130 may be a thin plate, which facilitates the subject to wear the measuring instrument 1100, and as a result the body 1130 is less likely to give the subject a sense of discomfort when the subject wears the measuring instrument 1100.

As illustrated in FIGS. 13 and 14, the temperature sensor 240 is coupled to the body 1130. The temperature sensor 240 is configured to be energized to the mastoid part side of the subject when the measuring instrument 1100 is worn. For example, the temperature sensor 240 is coupled to the body 1130 via a spring, and may be configured to be energized to the mastoid part side by the elasticity of the spring. However, the temperature sensor 240 may be configured to be energized to the mastoid part side by the mechanisms other than the spring. The force with which the temperature sensor 240 is energized may be, for example, such that the subject wearing the measuring instrument 1100 does not feel pain. The force with which the temperature sensor 240 is energized may be such a degree that, for example, the end portion 241 of the temperature sensor 240 does not separate from the mastoid part.

The body 1130 includes a connector 1150 to/from which a sensor capable of measuring the biological information of the subject can be attached/detached. The connector 1150 may be configured as, for example, a female connector. The connector 1150 may have a shape conforming to a predetermined standard. A measuring device capable of measuring the predetermined biological information may be connected to the connector 1150, for example, according to the state of the subject to be measured (or estimated) by the measuring instrument 1100. The followings describe an aspect in which the biosensor 210 is connected to the connector 1150.

FIG. 15 is a schematic external perspective view illustrating the measuring instrument 1100 in a state where the biosensor 210 is connected to the connector 1150. As illustrated in FIG. 15, the biosensor 210 may be connected to the cable 211, and the cable 211 may be connected to the connector 212. The cable 211 couples the biosensor 210 and the connector 212. The connector 212 allows the biosensor 210 to connect to the connector 1150 of the measuring instrument 1100. For example, the connector 212 may be configured as a male connector that can be attached to and detached from the connector 1150 of the measuring instrument 1100, for example.

The biosensor 210 may be configured so as to be worn on the measured part by sandwiching the measured part, for example. The biosensor 210 is configured so as to be worn on the ear lobe by sandwiching the ear lobe, which is a measured part, for example. The subject may measure the biological information by using the ear lobe of the auricle on the opposite side of the temperature sensor 240, for example, as the measured part. When the measuring instrument 1100 illustrated in FIGS. 13-16 is used, the biosensor 210 can acquire the biological information from the right ear lobe as a measured part. Further, the biosensor 210 may acquire the biological information from the left ear lobe as a measured part or from both right and left ear lobes as measured parts.

With reference to FIGS. 13 and 14 again, the coupling portion 1120 may include a battery holder 1121 in a part thereof. The battery holder 1121 includes a battery configured to drive various functional parts of the measuring instrument 1100.

The body 1130 and the battery may be disposed so that the force applied to the right auricle from the first wearing portion 1110R and the force applied to the left auricle from the second wearing portion 1110L will be substantially equal in the coupling portion 1120. That is, the body 1130 and the battery may be disposed in the coupling portion 1120 so that the left and right weight balances are substantially equal when the measuring instrument 1100 is worn. Here, "substantially equal" includes a range in which the subject wearing the measuring instrument 1100 does not feel uncomfortable with respect to the weight balance. In other words, approximately equal includes the range in which the subject does not feel that the left and right weights are not balanced when wearing the measuring instrument 1100. The body 1130 and the battery may be disposed at corresponding positions on the left and right, for example, in the coupling portion 1120. In the examples illustrated in FIGS. 13 and 14, the battery is disposed in the vicinity of the first wearing portion 1110R, and the body 1130 is disposed in the vicinity of the second wearing portion 1110L.

The measuring instrument 1100 according to this embodiment further includes a notification interface 260 that allows a subject wearing the measuring instrument 1100 to hear sound. In this embodiment, the notification interface 260 is provided on the end side of the wearing portion 1110, which is not coupled to the coupling portion 1120. In this embodiment, the notification interface 260 is configured to be disposed at the temple or above the ear of the subject when the subject wears the measuring instrument 1100. The notification interface 260 may be composed of a so-called bone conduction speaker that allows the subject to hear sound by transmitting vibration to the human body, for example. In this case, the notification interface 260 vibrates based on a control signal from the controller 250 included in the body 1130, for example. The vibration of notification interface 260 propagates to the subject's skull and thus the subject can hear the sound. In this case, as illustrated in FIG. 16, since the measuring instrument 1100 can allow the subject to hear the sound without covering the subject's ears, the subject can hear the surrounding sound. The notification interface 260 may be configured to be disposed at any position on the body such as the subject's temporal region, forehead or other head, neck, etc. when the subject wears the measuring instrument 1100.

However, the notification interface 260 does not necessarily have to be composed of a bone conduction speaker. The notification interface 260 may be composed of a device that transmits sound to the user by air vibration, for example, an earphone or a speaker. In this case, the notification interface 260 outputs sound based on a control signal from the controller. Further, in this disclosure, a bone conduction speaker and a device that transmits sound to the user by air vibration may be used together. That is, the notification interface 260 may be any combination of a bone conduction speaker and a device that transmits sound to the user by air vibration.

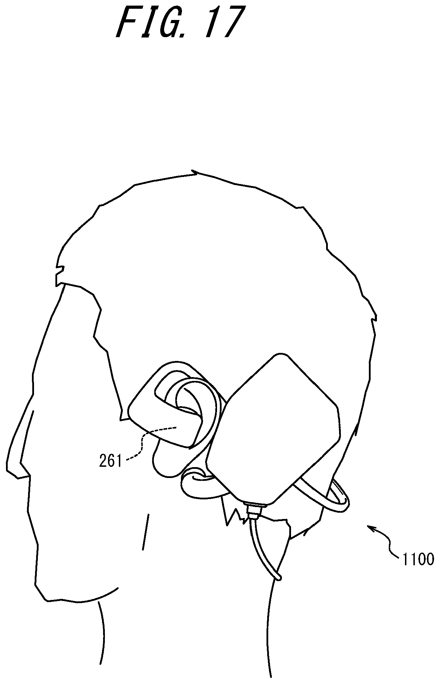

FIG. 17 is a diagram illustrating a state where the subject wears the measuring instrument 1100 when the notification interface 261 is configured by earphones, speakers, or the like. The measuring instrument 1100 illustrated in FIG. 17 has the same configuration as the measuring instrument 1100 illustrated in FIG. 16 except that the notification interface 260 is changed to the notification interface 261 in the measuring instrument 1100 illustrated in FIG. 16. As described above in FIG. 16, when the notification interface 260 is configured by a bone conduction speaker, it is not necessary to cover the subject's ears when the subject hears the sound. On the other hand, when the notification interface 261 is configured by an earphone, a speaker, or the like, as illustrated in FIG. 17, the part where the sound is output in the notification interface 261 may be brought close to the auricle or the external ear canal hole of the subject. For example, when the notification interface 261 is configured by a speaker, the part where the sound is output in the notification interface 261 may be configured to abut any position of the auricle of the subject (for example, near the external ear canal hole). Further, when the notification interface 261 is configured by earphones, for example, at least a part of the sound output portion (for example, earpiece) in the notification interface 261 may be configured to be inserted into the external ear canal hole of the subject. When the notification interface 261 is configured by earphones, speakers, or the like as illustrated in FIG. 17, the subject is less likely to hear surrounding sounds, but is more likely to be focused on the sound output from the notification interface 261.

Further, in examples illustrated in FIGS. 13 and 14, the notification interface 260 is provided on the end side of each of the first wearing portion 1110R and the second wearing portion 1110L. However, the notification interface 261 may be provided on the end side of only one of the first wearing portion 1110R and the second wearing portion 1110L, or at any position of the measuring instrument 1100 other than the end side of the wearing portion.

Further, in the measuring instrument 1100 according to an embodiment, the notification interface 160, the notification interface 660, the notification interface 260 and the notification interface 261 can notify at least one of the above described measurement results such as, for example, the SpO₂, the blood flow amount and the body temperature of the subject. Further, the notification interfaces 160, 660, 260 and 261 may notify the information relating to the likelihood that the subject may be in the state like cyanosis, for example.

### REFERENCE SIGNS LIST

- 100, 200, 400: Measurement apparatus
- 110, 210, 410: Biosensor
- 121, 221, 421: First laser light source
- 122, 222, 422: Second laser light source
- 131, 431: First light receiver
- 132, 432: Second light receiver
- 140, 240, 440: Temperature sensor
- 150, 250, 450, 550, 650: Controller
- 151, 251, 451, 551, 651: Processor
- 160, 260, 261, 460, 660: Notification interface
- 170, 270, 470, 570, 670: Memory
- 211: Cable
- 212: Connector
- 230: Light receiver
- 241: End portion
- 300: Measurement system
- 480, 580, 680: Communication interface
- 500: Information processor
- 600: Terminal apparatus
- 680: Input interface
- 700: Cerebral blood flow amount meter
- 800: Blood-pressure gauge
- 900: Thermometer
- 1000: Measuring instrument
- 1001: Holder
- 1002: Head band
- 1003: Connection
- 1100: Measuring instrument
- 1110: Wearing portion
- 1120: Coupling portion
- 1121: Battery holder
- 1130: Body
- 1150: Connector

## Claims

1. A measurement apparatus, comprising:
a first measurement unit configured to measure at least one of percutaneous oxygen saturation (SpO₂) and blood flow amount of a subject; and
a controller configured to estimate health condition of the subject based on information including at least one of a measured value of the SpO₂ and a measured value of the blood flow amount.

2. The measurement apparatus according to claim 1, comprising a second measurement unit configured to measure body temperature of the subject, wherein the controller estimates health condition of the subject based on information including a measured value of the body temperature.

3. The measurement apparatus according to claim 1 or 2, wherein the controller calculates respiratory rate of the subject based on at least one of the measured value of the SpO₂ and the measured value of the blood flow amount and estimates health condition of the subject based on information including the respiratory rate.

4. The measurement apparatus according to any one of claims 1 to 3, wherein the controller calculates Perfusion Index (PI) of the subject based on at least one of the measured value of the SpO₂ and the measured value of the blood flow amount and estimates health condition of the subject based on information including the PI.

5. The measurement apparatus according to any one of claims 1 to 4, comprising a notification interface configured to notify an estimation result by the controller.

6. The measurement apparatus according to claim 5, wherein the controller controls the notification interface to notify an estimation of health condition of the subject when at least one of the measured value of the SpO₂, the measured value of the blood flow amount, the measured value of the body temperature, a calculated value of the respiratory rate and a calculated value of the PI is lower than respective corresponding predetermined values.

7. The measurement apparatus according to any one of claims 1 to 6, wherein the controller estimates cyanosis of the subject.

8. A measurement method, comprising:
a measurement step of measuring at least one of percutaneous oxygen saturation (SpO₂) and blood flow amount of a subject; and
an estimation step of estimating health condition of the subject based on information including at least one of a measured value of the SpO₂ and a measured value of the blood flow amount.

9. A measurement program configured to cause a computer to execute:
a measurement step of measuring at least one of percutaneous oxygen saturation (SpO₂) and blood flow amount of a subject; and
an estimation step of estimating health condition of the subject based on information including at least one of a measured value of the SpO₂ and a measured value of the blood flow amount.
